(19) Europäisches Patentamt
European Patent Office
Office européen des brevets

(11) **EP 3 540 631 A1**

(12) **EUROPÄISCHE PATENTANMELDUNG**

(43) Veröffentlichungstag:
**18.09.2019 Patentblatt 2019/38**

(51) Int Cl.:
*G06K 9/00* (2006.01)  *G06K 9/62* (2006.01)
*G01N 33/49* (2006.01)

(21) Anmeldenummer: **18162033.7**

(22) Anmeldetag: **15.03.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder:
• **Richter, Lukas, Dr.**
**96114 Hirschaid (DE)**
• **Ugele, Matthias**
**92318 Neumarkt (DE)**
• **Hayden, Oliver, Dr.**
**85368 Moosburg (DE)**
• **Schmidt, Oliver, Dr.**
**91052 Erlangen (DE)**
• **Stanzel, Manfred, Dr.**
**92334 Berching (DE)**
• **Weniger, Markus, Dr.**
**90409 Nürnberg (DE)**

(54) **IN-VITRO-VERFAHREN ZUM MARKIERUNGSFREIEN BESTIMMEN EINES ZELLTYPS EINER WEISSEN BLUTZELLE**

(57) Die Erfindung betrifft ein in-vitro Verfahren zum markierungsfreien Bestimmen eines Zelltyps einer weißen Blutzelle in einer biologischen Probe, wobei eine Mikroskopiervorrichtung die Zelle abbildet und aus der Abbildung der Zelle mittels einer automatischen Bildanalyse physikalische Parameter der Zelle ermittelt werden, wobei anhand der physikalischen Parameter und anhand von Hauptkomponentenanalyse-Parametern (PCA-Parametern) der Zelltyp der weißen Blutzelle bestimmt wird, wobei die Hauptkomponentenanalyse-Parameter Linearkombinationen wenigstens eines Teils der physikalischen Parameter umfassen.

FIG 13

EP 3 540 631 A1

**Beschreibung**

Die Erfindung betrifft ein in-vitro Verfahren zum markierungsfreien Bestimmen eines Zelltyps einer weißen Blutzelle in einer biologischen Probe unter Verwendung einer Mikroskopiervorrichtung.

Das Bestimmen von Zellen und deren Zuordnung zu einem Zelltyp ist von großer Bedeutung in der Zytologie. In beispielsweise einer hämatologischen Untersuchung werden zelluläre Blutkomponenten, also Erythrozyten, Thrombozyten und weiße Blutzellen, bestimmt und quantifiziert. Eine Ermittlung der Leukozytenanzahl ("White Blood Count", WBC) sollte für eine umfangreiche Diagnostik die wesentlichen Populationen der weißen Blutzellen differenzieren können. Die weißen Zellen werden differenziert nach granulären Zellen (Neutrophilen, Eosinophilen, Basophilen) und nicht-granulären Zellen (Lymphozyten, Monozyten). Neben der Granularität unterscheiden sich die Zellen auch hinsichtlich der Fragmentierung des Zellkerns (ohne Fragmentierung: Mononukleare Zellen, also Lymphozyten und Monozyten; polymorphnukleare Zellen: Eosinophile, Basophile und Neutrophile) und der Zellgröße. Für die Differenzierung der granulären Zellen, im Besonderen eosinophile und basophile Granulozyten, wird eine Färbung eingesetzt. Die Auswertung der Zellpopulationen erfolgt üblicherweise durch vollautomatisierte Hämatologie-Analysatoren oder durch Mikroskopie. Vollautomatisierte Analysatoren müssen die Populationen nach festgesetzten Algorithmen analysieren (mit Hilfe von z.B. Impedanz-, Streulicht- und Absorptionsmessungen). Dies führt jedoch oft dazu, dass zum Beispiel bei pathologischen Proben Fehlermeldungen angezeigt werden. Im nächsten Schritt erfolgt üblicherweise eine Mikroskopie als Validierungsmethode für durch den Hämatologie-Analysator fehlerhaft bestimmte Zellen. Dieser Schritt ist aufwendig und kostenintensiv, da er neben Probenvorbereitung, Mikroskopie und weiteren manuell durchführbaren Arbeiten auch eine manuelle Bewertung erfordert.

Blutbildveränderungen sind ein Begleitphänomen einer Vielzahl von Erkrankungen und leisten deshalb einen wichtigen differentialdiagnostischen Beitrag. Das kleine Blutbild ist eine der häufigsten Laboruntersuchungen und umfasst u. a. die Hämoglobinbestimmung und die Ermittlung der Zellzahlen von Erythrozyten, Leukozyten und Thrombozyten. Das große Blutbild beinhaltet zusätzlich das Differentialblutbild, zu dem auch die Leukozytendifferenzierung gehört.

Aufgrund der verwendeten Methoden (z.B. Mie scattering) ist insbesondere für die Analyse der weißen Blutzellen eine aufwendige Probenvorbereitung (Zellen werden angefärbt bzw. partiell lysiert) nötig. Im Falle von pathologischen Blutproben erfolgt die Probenpräparation, wie z.B. die Lyse, jedoch oft nur unzureichend und deshalb ist eine manuelle Untersuchung der Blutzellen durch z.B. Ausstrich und Färbung notwendig. Diese Methode ist jedoch nur qualitativ und kann aufgrund der geringen Zahl an ausgezählten Zellen (etwa 100 bis 200 Zellen pro Slide) für seltene weiße Blutzellen nur eingeschränkt verwendet werden. Für die Erstdiagnose pathologischer Proben wie z.B. Leukämien ist der Blutausstrich mit anschließender Färbung der Blutzellen jedoch momentan die Standardmethode.

Die mechanisierte Leukozytendifferenzierung im Vollblut kann z.B. mittels Widerstandsmessung, Konduktivmessung, Laser-Streulichtmessung, Durchflusszytometrie oder zytochemischer Peroxidasereaktion erfolgen. Dabei werden zunächst die Erythrozyten lysiert und die Leukozyten in einer Durchflusszelle vereinzelt.

Um z.B. eine quantitative Blutzelldiagnostik durchzuführen, kann auf einen Hämatologie-Analysator verzichtet werden und stattdessen jede Einzelzelle mikroskopiert werden. Dies erlaubt es, das Blutbild unabhängig von festgesetzten Auswertealgorithmen und Flags quantitativ zu bestimmen. Nachteil dieses Ansatzes ist jedoch der geringere Probendurchsatz als bei einem Hämatologie-Analysator und der nach wie vor bestehende Aufwand, die Zellen auf Objektträgern zu immobilisieren und zu färben. Diese Färbungen haben zudem nur eine eingeschränkte Reproduzierbarkeit und zeigen eine hohe Abhängigkeit von Luftfeuchte, Färbungsdauer, Temperatur und mehr. Nachteil bei der reinen Mikroskopie ist es, die Zellvolumina zu quantifizieren im Vergleich zu Durchflusszytometern eingesetzt bei Hämatologie-Analysatoren.

Für die Erstellung eines manuellen Blutbildes werden spezielle, auf die Analyse von Vollblut ausgelegte Färbetechniken, wie z. B. die Wright-Giemsa Färbung, verwendet. Dazu wird Vollblut auf einem Objektträger ausgestrichen und zur Erstellung eines Differentialblutbildes, beispielsweise etwa 100 Leukozyten, in schwierigen Fällen gegeben falls etwa 3 mal 100 Zellen, ausgezählt.

Für die Erstdiagnose pathologischer Proben ist der Blutausstrich mit anschließender Färbung der Blutzellen die Standardmethode. Bei Leukämien können so anhand des Differentialblutbildes durch charakteristisch vorkommende Zellpopulationen folgende Arten unterschieden werden: "akute myeloische Leukämie (AML)", "akute lymphatische Leukämie (ALL)", "chronische myeloische Leukämie (CML)" und "chronische lymphatische Leukämie (CLL)".

Aufgrund der geringen Statistik bei Blutausstrichen (etwa 100 bis 200 Zellen pro Slide) wird zu Verifizierung der Diagnose sowie zur genauen Bestimmung von Untertypen von Leukämien in der Regel Durchflusszytometrie verwendet. Dabei werden die weißen Blutzellen mit speziellen (Fluoreszenz-) Antikörpern markiert und die Differenzierung erfolgt über charakteristische Expressionsmuster die sich im Streubild wiederspiegelt. Zusätzlich kann bei komplizierten Fällen für eine genaue Diagnose eine Knochenmarkspunktion erforderlich sein.

Der Einsatz von Farbstoffen zur Differenzierung von verschiedenen (Blut-) Zellen wurde bereits in den 60er Jahren des 19. Jahrhunderts entwickelt. Gustav Giemsa entwickelte die nach ihm benannte Färbung ursprünglich zur Sichtbarmachung von Malaria-Parasiten und anschließend modifiziert zur Färbung von Treponema pallidum, dem Erreger der Syphilis. Die heute verwendete Wright-Giemsa Färbung erlaubt die Unterscheidung von weißen Blutzellen und stellt deshalb die Erstdiagnose für leukämische Erkrankungen dar.

Für eine genaue Bestimmung des Leukämie Immuno-Phänotyps wird standardmäßig Durchflusszytometrie (fluorescence-activated cell sorting, FACS) verwendet. Hierbei wird eine Vielzahl von verschiedenen (Fluoreszenz-) Antikörpern verwendet, als Proben werden sowohl peripheres Blut als auch Knochenmarkproben Verwendet. Die Auswertung erfolgt mittels speziellen Programmen zur Analyse von FACS Daten (z.B. Kaluza). Dabei kann durch das Vorhandensein von Datenpunkten in vorher festgelegten Bereichen, die auch als "Gates" bezeichnet werden, eine Unterscheidung von unterschiedlichen Krankheitsbildern durchgeführt werden. Eine färbungs-/markierungsfreie Unterscheidung der vier oben genannten Leukämieformen mit einer rein mikroskopischen Methode ist bisher nicht bekannt.

Aus der US 8406498 ist ein bildgebendes Duchlasszytometer bekannt, wodurch die Differenzierung der weißen Blutkörper direkt bestimmt werden kann. Auch in diesem Fall kommt jedoch eine Färbung zum Einsatz, um insbesondere eosinophile und basophile Granulozyten zu unterscheiden. Somit ergeben sich auch hier die oben genannten Nachteile.

Eine der Erfindung zugrundeliegende Aufgabe ist folglich das Bereitstellen eines effizienteren und robusteren Verfahrens zum Differenzieren von weißen Blutzellen, bei dem insbesondere eine Färbung und/oder Markierung der Blutzellen nicht notwendig ist.

Die Aufgabe wird von dem erfindungsgemäßen Verfahren, der erfindungsgemäßen Zellanalyseeinrichtung und der Mikroskopiervorrichtung gemäß den unabhängigen Patentansprüchen gelöst. Vorteilhafte Weiterbildungen der Erfindung sind durch die Unteransprüche gegeben.

Das erfindungsgemäße in-Vitro-Verfahren zum markierungsfreien Bestimmen eines Zelltyps einer Blutzelle in einer biologischen Probe wird mit Hilfe einer Mikroskopiervorrichtung und einer Zellanalyseeinrichtung durchgeführt. Eine Mikroskopiervorrichtung ist dabei ein Gerät, mit dem sehr kleine Objekte stark vergrößert betrachtet werden können, und umfasst beispielsweise ein Lichtmikroskop, ein Rasterelektronenmikroskop, ein Phasenkontrastmikroskop, ein digitales holographisches Mikroskop oder ein Mikroskop mit einem Ultraschallsensor (z.B. ein akustisches Mikroskop). Die Mikroskopiervorrichtung bildet dabei die Zelle ab. Aus der Abbildung der Zelle werden mittels einer automatischen Bildanalyse physikalische Parameter der Zelle ermittelt.

Die Zellanalyseeinrichtung, also ein Gerät oder eine Gerätekomponente, die zur elektronischen Datenverarbeitung geeignet ist und dazu eingerichtet ist, aus der Abbildung der Zelle mittels einer automatischen Bildanalyse physikalische Parameter der Zelle zu ermittelt, führt ein wie folgt näher beschriebenes erfindungsgemäßes Verfahren durch.

Gegenstand der Erfindung ist ein in-vitro Verfahren zum markierungsfreien Bestimmen eines Zelltyps einer weißen Blutzelle in einer biologischen Probe, wobei eine Mikroskopiervorrichtung die Zelle abbildet und aus der Abbildung der Zelle mittels einer automatischen Bildanalyse physikalische Parameter der Zelle ermittelt werden, wobei anhand der physikalischen Parameter und anhand von Hauptkomponentenanalyse-Parametern (PCA-Parametern) der Zelltyp der weißen Blutzelle bestimmt wird, wobei die Hauptkomponentenanalyse-Parameter Linearkombinationen wenigstens eines Teils der physikalischen Parameter umfassen.

Bevorzugt wird der Zelltyp der weißen Blutzelle anhand von vorbestimmten Kriterien bezüglich der physikalischen Parameter und der Hauptkomponentenanalyse-Parameter (PCA-Parameter) bestimmt, wobei wenn die jeweiligen vorbestimmten Kriterien erfüllt sind, der entsprechende Zelltyp vorliegt.

Der Begriff "vorbestimmte Kriterien" bezieht sich auf ein Kriterium oder mehrere Kriterien, welche aufgrund von einem oder mehreren physikalischen und/oder Hauptkomponentenanalyse-Parametern (PCA-Parametern) bestimmt wird. Das jeweilige Kriterium wird dabei bevorzugt bestimmt auf der Basis eines Vergleichs zwischen entsprechenden Abbildungen von verschiedenen, zu unterscheidenden weißen Blutzelltypen.

Das Verfahren erlaubt aufgrund des Verzichts auf ein Färbeverfahren einen hohen Probendurchsatz und eine qualitative und/oder quantitative Bestimmung von Zellen. Dadurch wird eine Mikroskopie mit einem hohem Probendurchsatz ermöglicht und es kann auf herkömmliche Hämatologie-Analysatoren verzichtet werden.

Bevorzugt handelt es sich bei dem bestimmten Zelltyp der weißen Blutzelle um einen der folgenden Haupttypen von weißen Blutzellen aus der Gruppe umfassend Monozyten, neutrophile Granulozyten, basophile Granulozyten, eosinophile Granulozyten und Lymphozyten.

In einer weiteren bevorzugten Ausführung des erfindungsgemäßen Verfahrens handelt es sich bei dem bestimmten Zelltyp um einen der Haupttypen von weißen Blutzellen und/oder einem Subtyp von weißen Blutzellen aus der Gruppe umfassend Myelozyten, Metamyelozyten, Promyelozyten, Blasten, Megakaryozyten, Plasmazellen, atypische Lymphozyten und Sezary Zellen.

In einer weiteren bevorzugten Ausführungsform werden anhand der Bestimmung des jeweiligen Zelltyps einer Vielzahl von weißer Blutzellen in einer Probe die jeweiligen Zellpopulationen charakterisiert und anhand der vorliegenden Zellpopulationen bestimmt, ob eine akute myeloische Leukämie (AML), eine akute lymphatische Leukämie (ALL), eine chronische myeloische Leukämie (CML) oder eine chronische lymphatische Leukämie (CLL) in dem Patient, von dem die Probe stammt, vorliegt.

Bevorzugt umfassen die physikalischen Parameter der Zelle Parameter aus der folgenden Gruppe umfassend umfasste Fläche der Zelle (cellArea), Umfang der Zelle (perimeter), Breite der Zelle (width), Höhe der Zelle (height), Verhältnis von Breite und Höhe der Zelle (aspectRatio), Ähnlichkeit der geometrischen Form der Zelle mit einem Kreis (circularity), durchschnittlicher Radius der Zelle (radiusMean), Varianz des Radius der Zelle (radiusVariance), Bedeckungsgrad der Zelle (solidity), äquivalenter Durchmesser entsprechend der umfassten Fläche der Zelle (equivalentDiameter), optisches Volumen der Zelle (opticalVolume), maximale optische Höhe der Zelle (opticalHeightMaximum), minimale optische Höhe der Zelle (opticalHeightMinimum), durchschnittliche optische Höhe der Zelle (opticalHeightMean), Varianz der optischen Höhe der Zelle (opticalHeightVariance), Bikonkavität der Zelle (biconcavity), Sphärizität der Zelle (sphericity), Verschiebung des Massenschwerpunkts der Zelle (massCenterShift), Kontrast der Zelle (contrast), Verschiedenheit der Zelle (dissimilarity), Homogenität der Zelle (homogeneity), Energie der Zelle (energy), Entropie der Zelle (entropy).

Bevorzugt sind die physikalischen Parameter der Zelle wie folgt definiert.

| Bezeichnung | Einheit | Definition/Bemerkungen |
| --- | --- | --- |
| cellArea | $\mu m^2$ | Contour area |
| perimeter | $\mu m$ | Contour perimeter |
| width | $\mu m$ | Width of rotated bounding rectangle with minimum area |
| height | $\mu m$ | Height of rotated bounding rectangle with minimum area. |
| aspectRatio | | max (width,height) /min (width, height) ; |
| circularity | [0,1] | $4\pi$* cellArea / perimeter2. Circularity of a circle is 1. The closer it gets to 0, the less circular the contour is. |
| radiusMean | $\mu m$ | The mean distance between the centroid of the contour and each contour support point. Centroid is calculated out of contour moments. Please note that for contours defined by only a few points this does not match the radius for each contour pixel, but only for the supporting points (e.g. a rectangle can be defined by four supporting points), speeding up computation. |
| radiusVariance | | The variance of the distance between the centroid of the contour and each contour support point. |
| solidity | | Solidity is the ratio of contour area to its convex hull area. First, the convex hull of the contour is calculated. The solidity is calculated by cellArea / convex hull area. Solidity grows for irregular shaped objects. |
| equivalentDiameter | $\mu m$ | Equivalent Diameter is the diameter of the circle whose area is same as the contour area. Calculated by sqrt((4*cellArea) /$\pi$). |
| opticalVolume | $\mu m^3$ | The volume of a single pixel can be calculated by phase value * 0.53 (wavelength) / (2 $\pi$) * 0,345 (pixel size). The overall optical volume is the sum of all contour volume pixels. |
| opticalHeightMaximum | | The maximum phase value inside the contour. |
| opticalHeightMinimum | | The minimum phase value inside the contour. |
| opticalHeightMean | | The mean phase value inside the contour. |
| opticalHeightVariance | | The phase value variance inside the contour. |
| biconcavity | [-1,1] | Extracts pixel phase values of a horizontal and vertical cut through the middle of the contours bounding rectangle. Checks correlation of pixel values with the polynominal - 4*x^4 + 4*x^2 + 0,5 scaled to the same length. Return minimum value of the two cuts. |
| sphericity | [-1,1] | Extracts pixel phase values of a horizontal and vertical cut through the middle of the contours bounding rectangle. Checks correlation of pixel values with the polynom -x^2 + 1 scaled to the same length. Return minimum value of the two cuts. |

(fortgesetzt)

| Bezeichnung | Einheit | Definition/Bemerkungen |
|---|---|---|
| massCenterShift | | Euclidian distance between geometric centroid and mass centroid. Mass centroid is calculated out of the squared phase values. Values for X and Y are calculated by getting the 1D mass center along each axis. Axis mass center is calculated by (e.g. for x axis) mc = 1 / (m1 + ... + mn) * (x1*m1 + ... + xm*mn) with the ml...mn the summed-up phase values for each x value. |
| contrast | | 5 features are extracted out of the gray level co-occurrence matrix. |
| | | To speed up the process, the gray values are scaled by ¼ before constructing the matrix (resulting in only 64 possible values). Contrast or local intensity variation will favor contributions from P(i,j) away from the diagonal. |
| dissimilarity | | Dissimilarity measure based on GLCM of phase values converted to 6bit grayscale image for the pixels inside cell contour. |
| homogenity | | homogeneous scene will contain only a few gray levels, giving a GLCM with only a few but relatively high values of P(i,j) |
| energy | | Measures order. |
| entropy | | Measures disorder. |

Details to the definition of contrast and the 5 features which are extracted out of the gray level co-occurrence matrix can be found in "GLCM Texture: A Tutorial v. 3.0 March 2017" at the webpages of the University of Calgary under http://www.fp.ucalgary.ca/mhallbey/tutorial.htm (Uri http://hdl.handle.net/1880/51900).

In weiteren bevorzugten Ausführungsformen können zusätzliche Informationen erfasst werden, welche in Abhängigkeit des Mirkroskopieverfahrens zusätzlich zur Verfügung stehen. Beispielsweise kann die Intensitätsverteilung, ein Polarisations- oder Fluoreszenzkontrast zusätzlich genutzt werden um eine bessere Differenzierung zu ermöglichen.

Bevorzugt umfassen die Hauptkomponentenanalyse-Parameter (PCA-Parameter) eins, zwei, drei vier oder fünf verschiedene Linearkombinationen von Parametern.

Bevorzugt umfassen die Hauptkomponentenanalyse-Parameter (PCA-Parameter) Linearkombinationen der folgenden Parameter.

Wenigstens ein PCA-Parameter umfasst bevorzugt eine Linearkombination der Parameter cellArea, perimeter, width, height, aspectRatio, circularity, radiusMean, radiusVariance, solidity, equivalentDiameter, opticalVolume, opticalHeightMaximum, opticalHeightMinimum, opticalHeightMean, opticalHeightVariance, biconcavity, sphericity, massCenterShift, contrast, dissimilarity, homogenity, energy, entropy.

Besonders umfassen die PCA-Parameter insgesamt sechs PCA-Parameter, wobei jeder der PCA-Parameter jeweils bevorzugt eine Linearkombination der Parameter cellArea, perimeter, width, height, aspectRatio, circularity, radiusMean, radiusVariance, solidity, equivalentDiameter, opticalVolume, opticalHeightMaximum, opticalHeightMinimum, opticalHeightMean, opticalHeightVariance, biconcavity, sphericity, massCenterShift, contrast, dissimilarity, homogenity, energy und entropy umfasst.

Bevorzugt umfassen die Hauptkomponentenanalyse-Parameter (PCA-Parameter) die folgenden Parameter Val1 bis Val6.

```
Val1=-0.2514235930087*((cell.cellArea-
(42.9755016744))/10.5788867263146)+-
0.249974748498971*((cell.perimeter-
(24.576753500494))/3.20109088487221)+-
0.237593744589169*((cell.width-
(7.35723468359436))/1.00790834623485)+-
0.241385243730167*((cell.height-
(7.28724864632538))/1.00167379952967)+0.0500690750767191*((ce
ll.aspectRatio-
(1.0727084035923))/0.0503940328943091)+-
0.0325934362800635*((cell.circularity-
(0.879203898319695))/0.0171374141190303)+-
0.251748682215606*((cell.radiusMean-
(3.67481430203961))/0.47807494523509)+-
```

0.0358686373697572*((cell.radiusVariance-(0.534334347573423))/0.135861132157575)+-0.0713429028935052*((cell.solidity-(0.973653367948167))/0.00642977966295229)+-0.251801959117612*((cell.equivalentDiameter-(7.33498993492049))/0.957125629695256)+-0.271859966910455*((cell.opticalVolume-(10.9434893272532))/4.39789306763487)+-0.264893256207984*((cell.opticalHeightMaximum-(4.0508608252182))/1.13386516864492)+-0.019499726282544*((cell.opticalHeightMinimum-(0.917628988325119))/0.00943812184922224)+-0.263403666244868*((cell.opticalHeightMean-(2.56913326007255))/0.578640046049587)+-0.264398202286343*((cell.opticalHeightVariance-(0.891494679587935))/0.319599313085186)+0.0683113521964872*((cell.biconcavity-(-0.1590427888386))/0.0878662330982137)+0.0532706264409643*((cell.sphericity-(0.952179340809924))/0.0556215659482945)+-0.135017569551957*((cell.massCenterShift-(0.321787377964238))/0.279787203971966)+-0.23775558906941*((cell.contrast-(5.99415440523299))/3.28737834908757)+-0.245223436384692*((cell.dissimilarity-(1.8261947255628))/0.524533027046986)+0.241198017846531*((cell.homogenity-(0.421196396914475))/0.0787508131258171)+0.258958179846163*((cell.energy-(0.106234099933335))/0.0303777835437848)+-0.267352762571249*((cell.entropy-(4.85017551257248))/0.552270252441689)+0;

Val2=-0.168670616411677*((cell.cellArea-(42.9755016744))/10.5788867263146)+-0.205776175336618*((cell.perimeter-(24.576753500494))/3.20109088487221)+-

0.186558934231474*((cell.width-(7.35723468359436))/1.00790834623485)+-0.183141003655306*((cell.height-(7.28724864632538))/1.00167379952967)+-0.0928942621464152*((cell.aspectRatio-(1.0727084035923))/0.0503940328943091)+0.505899790631485*((cell.circularity-(0.879203898319695))/0.0171374141190303)+-0.169985535864666*((cell.radiusMean-(3.67481430203961))/0.47807494523509)+-0.377167185427535*((cell.radiusVariance-(0.534334347573423))/0.135861132157575)+0.416007969970529*((cell.solidity-(0.973653367948167))/0.00642977966295229)+-0.166991998607427*((cell.equivalentDiameter-(7.33498993492049))/0.957125629695256)+-0.00731744872935898*((cell.opticalVolume-(10.9434893272532))/4.39789306763487)+0.106456651440064*((cell.opticalHeightMaximum-(4.0508608252182))/1.13386516864492)+0.20085391219078*((cell.opticalHeightMinimum-(0.917628988325119))/0.00943812184922224)+0.122345079558179*((cell.opticalHeightMean-(2.56913326007255))/0.578640046049587)+0.101062869417052*((cell.opticalHeightVariance-(0.891494679587935))/0.319599313085186)+0.217089205338001*((cell.biconcavity-(-0.1590427888386))/0.0878662330982137)+-0.0619295694253695*((cell.sphericity-(0.952179340809924))/0.0556215659482945)+0.0156956887744252*((cell.massCenterShift-(0.321787377964238))/0.279787203971966)+0.198114038338057*((cell.contrast-(5.99415440523299))/3.28737834908757)+0.180425506176449*((cell.dissimilarity-(1.8261947255628))/0.524533027046986)+-0.135400526577736*((cell.homogenity-

```
(0.421196396914475))/0.0787508131258171)+-
0.0267942709719767*((cell.energy-
(0.106234099933335))/0.0303777835437848)+0.0722804026404807*(
(cell.entropy-
(4.85017551257248))/0.552270252441689)+0;

Val3=-0.152245658793807*((cell.cellArea-
(42.9755016744))/10.5788867263146)+-
0.137744345235438*((cell.perimeter-
(24.576753500494))/3.20109088487221)+-
0.139046382561896*((cell.width-
(7.35723468359436))/1.00790834623485)+-
0.136640064377995*((cell.height-
(7.28724864632538))/1.00167379952967)+0.143071973661924*((cel
l.aspectRatio-
(1.0727084035923))/0.0503940328943091)+-
0.311832846414789*((cell.circularity-
(0.879203898319695))/0.0171374141190303)+-
0.158405722299146*((cell.radiusMean-
(3.67481430203961))/0.47807494523509)+0.2147365796495*((cell.
radiusVariance-
(0.534334347573423))/0.135861132157575)+-
0.290348831318269*((cell.solidity-
(0.973653367948167))/0.00642977966295229)+-
0.160436060932553*((cell.equivalentDiameter-
(7.33498993492049))/0.957125629695256)+-
0.0283231756746574*((cell.opticalVolume-
(10.9434893272532))/4.39789306763487)+0.108904826169979*((cel
l.opticalHeightMaximum-(4.0508608252182))/1.13386516864492)+-
0.0541041915771913*((cell.opticalHeightMinimum-
(0.917628988325119))/0.00943812184922224)+0.0482898928982701*
((cell.opticalHeightMean-
(2.56913326007255))/0.578640046049587)+0.0740565216581285*((c
ell.opticalHeightVariance-
(0.891494679587935))/0.319599313085186)+0.33268062359036*((ce
ll.biconcavity-(-
```

```
0.1590427888386))/0.0878662330982137)+-
0.486496428597712*((cell.sphericity-
(0.952179340809924))/0.0556215659482945)+0.404071847572092*((
cell.massCenterShift-
(0.321787377964238))/0.279787203971966)+0.200214346124148*((c
ell.contrast-
(5.99415440523299))/3.28737834908757)+0.168546834749071*((cel
l.dissimilarity-
(1.8261947255628))/0.524533027046986)+-
0.114748435539571*((cell.homogenity-
(0.421196396914475))/0.0787508131258171)+-
0.0143576741936659*((cell.energy-
(0.106234099933335))/0.0303777835437848)+0.070606562249387*((
cell.entropy-
(4.85017551257248))/0.552270252441689)+0;


Val4=-0.261610677611746*((cell.cellArea-
(29.4395201580311))/5.11005121908855)+-
0.261094203446583*((cell.perimeter-
(20.4714469010994))/1.73763119886254)+-
0.222861676546857*((cell.width-
(6.12959751130435))/0.59548518687133)+-
0.229916753106545*((cell.height-
(6.05150421944159))/0.591609173525849)+0.011807520590896*((ce
ll.aspectRatio-
(1.0869912949338))/0.0546526376570488)+0.0262225683553752*((c
ell.circularity-
(0.876941946272043))/0.0179521439605036)+-
0.261160667395425*((cell.radiusMean-
(3.05876819557268))/0.255900843697689)+-
0.0941868494695694*((cell.radiusVariance-
(0.516064433989357))/0.111459058693624)+-
0.0110718002570505*((cell.solidity-
(0.971597800798176))/0.00720404258117219)+-
0.260831711849364*((cell.equivalentDiameter-
(6.10101233363755))/0.511139800830794)+-
0.283417789826245*((cell.opticalVolume-
(5.49637143295321))/1.39356683120867)+-
```

```
0.28041071625337*((cell.opticalHeightMaximum-
(2.78997632108204))/0.357459556742829)+0.00345191681990364*((
cell.opticalHeight
Minimum-(0.916982544759395))/0.0114607243403515)+-
0.267687084654929*((cell.opticalHeightMean-
(1.90610826785609))/0.176319079561605)+-
0.279755464234688*((cell.opticalHeightVariance-
(0.531938907690763))/0.107019179729975)+0.121035300134979*((c
ell.biconcavity-(-
0.11874291555068))/0.0878957408234967)+-
0.0161513556541555*((cell.sphericity-
(0.955634909899621))/0.04264168644697)+-
0.107830413342139*((cell.massCenterShift-
(0.205413097350611))/0.143266493406051)+-
0.216912000881217*((cell.contrast-
(2.94739193186445))/0.852979848483421)+-
0.233161325461211*((cell.dissimilarity-
(1.31231869619681))/0.185666125635144)+0.22121616068744*((ce
ll.homogenity-
(0.499177299392145))/0.043929343763229)+0.237462579736086*((c
ell.energy-
(0.141919956703913))/0.0213959679195142)+-
0.268766115142943*((cell.entropy-
(4.20317134129727))/0.263391471017291)+0;


Val5=0.277185206648515*((cell.cellArea-
(29.4395201580311))/5.11005121908855)+0.289025861167001*((cel
l.perimeter-
(20.4714469010994))/1.73763119886254)+0.27869771372156*((cell
.width-
(6.12959751130435))/0.59548518687133)+0.226791977731385*((cel
l.height-
(6.05150421944159))/0.591609173525849)+-
0.0154016557869195*((cell.aspectRatio-
(1.0869912949338))/0.0546526376570488)+-
0.104962015198417*((cell.circularity-
(0.876941946272043))/0.0179521439605036)+0.280118171306254*((
cell.radiusMean-
```

```
(3.05876819557268))/0.255900843697689)+0.122071737825644*((ce
ll.radiusVariance-
(0.516064433989357))/0.111459058693624)+-
0.0757806415365649*((cell.solidity-
(0.971597800798176))/0.00720404258117219)+0.279776181994244*(
(cell.equivalentDiameter-
(6.10101233363755))/0.511139800830794)+0.127079667257615*((ce
ll.opticalVolume-
(5.49637143295321))/1.39356683120867)+-
0.166674686515488*((cell.opticalHeightMaximum-
(2.78997632108204))/0.357459556742829)+-
0.0801206102287044*((cell.opticalHeightMinimum-
(0.916982544759395))/0.0114607243403515)+-
0.13904585273397*((cell.opticalHeightMean-
(1.90610826785609))/0.176319079561605)+-
0.157320201651437*((cell.opticalHeightVariance-
(0.531938907690763))/0.107019179729975)+-
0.0137496049824385*((cell.biconcavity-
(-0.11874291555068))/0.0878957408234967)+-
0.0124754663656207*((cell.sphericity-
(0.955634909899621))/0.04264168644697)+-
0.00354707655665248*((cell.massCenterShift-
(0.205413097350611))/0.143266493406051)+-
0.337317104471527*((cell.contrast-
(2.94739193186445))/0.852979848483421)+-
0.351605489070963*((cell.dissimilarity-
(1.31231869619681))/0.185666125635144)+0.324115001671015*((ce
ll.homogenity-
(0.499177299392145))/0.043929343763229)+0.20286086484872*((ce
ll.energy-
(0.141919956703913))/0.0213959679195142)+-
0.208528317348662*((cell.entropy-
(4.20317134129727))/0.263391471017291)+0;

        Val6=-0.0822117013411024*((cell.cellArea-
(29.4395201580311))/5.11005121908855)+-
0.0136983441801879*((cell.perimeter-
(20.4714469010994))/1.73763119886254)+-
```

EP 3 540 631 A1

0.0421965767155267*((cell.width-
(6.12959751130435))/0.59548518687133)+-
0.0105097694602176*((cell.height-
(6.05150421944159))/0.591609173525849)+0.0829553453256781*((c
ell.aspectRatio-
(1.0869912949338))/0.0546526376570488)+-
0.601802580015178*((cell.circularity-
(0.876941946272043))/0.0179521439605036)+-
0.0832961277585592*((cell.radiusMean-
(3.05876819557268))/0.255900843697689)+0.312125036264253*((ce
ll.radiusVariance-
(0.516064433989357))/0.111459058693624)+-
0.548644911166917*((cell.solidity-
(0.971597800798176))/0.00720404258117219)+-
0.0887226111099722*((cell.equivalentDiameter-
(6.10101233363755))/0.511139800830794)+-
0.065558074724381*((cell.opticalVolume-
(5.49637143295321))/1.39356683120867)+0.00635200779277076*((c
ell.opticalHeight
Maximum-(2.78997632108204))/0.357459556742829)+-
0.208242751746043*((cell.opticalHeightMinimum-
(0.916982544759395))/0.0114607243403515)+-
0.052855467177894*((cell.opticalHeightMean-
(1.90610826785609))/0.176319079561605)+0.0139425503541623*((c
ell.opticalHeightVariance-
(0.531938907690763))/0.107019179729975)+0.0212708260463394*((
cell.biconcavity-(-
0.11874291555068))/0.0878957408234967)+-
0.281890905467496*((cell.sphericity-
(0.955634909899621))/0.04264168644697)+0.258391327626016*((ce
ll.massCenterShift-
(0.205413097350611))/0.143266493406051)+0.0744517932956948*((
cell.contrast-
(2.94739193186445))/0.852979848483421)+0.0587637354201565*((c
ell.dissimilarity-
(1.31231869619681))/0.185666125635144)+-
0.0404667518409852*((cell.homogenity-

13

```
(0.499177299392145))/0.043929343763229)+-
0.0159289744001*((cell.energy-
(0.141919956703913))/0.0213959679195142)+0.0247292136860108*(
(cell.entropy-
(4.20317134129727))/0.263391471017291)+0;
```

Bevorzugt umfasst die Abbildung der Zelle ein Überlagern einer Objektwelle mit einer Referenzwelle, ein Aufnehmen eines resultierenden Interferogramms und/oder eine computerimplementierte mathematische Rekonstruktion.

Besonders vorteilhaft ist dabei die Anwendung der digitalen holographischen Mikroskopie, da sie erlaubt die Phaseninformation des Objekts quantitativ zu erfassen. Gemäß einer weiteren Ausführungsform des erfindungsgemäßen Verfahrens wird die Abbildung der weißen Blutzelle daher mittels digitaler holographischer Mikroskopie, Interferenzphasenmikroskopie und/oder quantitativer Phasenmikroskopie ermittelt. Diese Mikroskopiemethoden ermöglichen eine besonders hohe axiale Auflösung, d.h. in Richtung der optischen Achse des Mikroskops. Eine digitale holographische Mikroskopie ermöglicht eine Auflösung von bis zu 1nm in z-Richtung. Dies entspricht einer Präzision die um einen Faktor 100-1000 höher liegt als mit anderen bekannten lichtmikroskopischen Verfahren (z.B. konfokales Mikroskop). Aufgrund der präzisen Ermittlung des Höhenprofils ist somit eine genauere Bestimmung des Zelltyps möglich.

Bevorzugt handelt es sich bei der Mikroskopiervorrichtung somit um ein Mikroskop zur Durchführung von digitaler holographischer Mikroskopie (DHM), Interferenzphasen-Mikroskopie oder quantitativer Phasen Mikroskopie.

Bevorzugt werden die weißen Blutzellen in einer Durchflusszelle von der Mikroskopiervorrichtung abgebildet, wobei die Durchflusszelle bevorzugt einen Kanal mit rechteckigem oder quadratischem Querschnitt umfasst.

Bevorzugt werden die weißen Blutzellen mittels laminarer Hüllströme, bevorzugt mittels vier laminarer Hüllströme, fokussiert.

Bevorzugt werden vor der Abbildung der weißen Blutzellen mittels der Mikroskopiervorrichtung rote Blutzellen mittels selektiver Lyse aus der Probe entfernt.

Ein weiterer Vorteil wird erzielt, wenn das Verfahren mit einer oder mehreren ungefärbten und/oder ungetrockneten Zellen durchgeführt wird. Da die Vitalität der Zellen durch das erfindungsgemäße Verfahren nicht beeinträchtigt wird, können die durch das Verfahren bestimmten Zellen nach Abschluss des Bestimmungsverfahrens für Folgeanalysen weiterverwendet werden.

Das erfindungsgemäße Verfahren kann gemäß einem weiteren Ausführungsbeispiel ein Phänotypisieren der Zelle mit einem Marker und/oder das Exprimieren eines vorbestimmten Rezeptors zum weiteren Zuordnen der Zelle umfassen. Dies ermöglicht eine weitergehende molekularbiologische Untersuchung der bestimmten Zelle.

Eine besondere Relevanz erhält das erfindungsgemäße Verfahren, wenn es gemäß einer weiteren Ausführungsform anhand einer Vollblutprobe durchgeführt wird und/oder die biologische Probe Blutzellen, Monozyten, neutrophile Granulozyten, basophile Granulozyten, eosinophile Granulozyten und/oder Lymphozyten enthält. Dies findet vor allem bei der Bestimmung eines Blutbildes in der Hämatologie Anwendung.

Ein weiterer Gegenstand der Erfindung ist eine Zellanalyseeinrichtung, zum Beispiel umfassend einen Mikrochip oder einen Mikrocontroller, die dazu eingerichtet ist, eine oder mehrere der oben beschriebenen Ausführungsformen des erfindungsgemäßes Verfahren durchzuführen.

Ein weiterer Gegenstand der Erfindung ist eine Mikroskopiervorrichtung zum markierungsfreien Bestimmen eines Zelltyps einer weißen Blutzelle einer biologischen Probe, umfassend eine erfindungsgemäße Zellanalyseeinrichtung.

Vorzugsweise umfasst die Mikroskopiervorrichtung ein digitales holographisches Mikroskop.

Gemäß einer weiteren Ausführungsform des erfindungsgemäßen Verfahrens kann das Bestimmen des weißen Blutzelltyps ein Bestimmen eines Zellstadiums der Zelle umfassen, insbesondere zum Differenzieren eines Zellalters, eines physiologischen oder morphologischen Zustands der Zelle oder eines Aktivitätszustands der Zelle. Dies ermöglicht ein Aufnehmen einer Bildsequenz von z.B. Aktivierungsvorgängen einer Zelle.

Die digitale holographische Mikroskopie (DHM), auch Interferenzphasenmikroskopie genannt, zeichnet sich dadurch aus, dass sie sowohl die Intensität als auch die Phase eines Objekts quantitativ in einer Aufnahme erfassen kann. Dafür wird nicht, wie in der Mikroskopie üblich, eine Intensitätsverteilung aufgenommen, welche sich durch Absorption und Streuung von Licht am Objekt ergibt, sondern eine Wellenfront, welche sich aus der Überlagerung einer Objekt- und einer Referenzwelle ergibt. Daraus kann mit Hilfe eines Computers die Intensitäts- und Phaseninformation des Objekts rekonstruiert werden.

Diese Mikroskopie-Methode eignet sich für die Untersuchung von biologischen Proben, da diese ohne weitere Probenvorbereitung im Wesentlichen transparent sind für sichtbares Licht und damit in einem herkömmlichen Mikroskop einen geringen Kontrast aufweisen. Durch die Aufnahme der Phaseninformation ist es möglich, die Morphologie der Zelle sehr präzise zu erfassen und mit Hilfe dieser Information eine Differenzierung der Zelle vorzunehmen.

Gegenüber herkömmlichen Phasenkontrastmikroskopen, welche üblicherweise durch einen Phasenring im Objektiv und eine Ringblende im Kondensor realisiert werden, bietet die DHM den Vorteil, dass die Phase quantitativ bestimmt werden kann. Entsprechend spricht man auch von quantitativer Phasenkontrastmikroskopie. Erst durch die Quantifizierung der Phase wird eine reproduzierbare Rekonstruktion des Höhenprofils des Objekts ermöglicht und erst dadurch kann eine automatisierte Bestimmung des Zelltyps erfolgen.

Es ist darauf hinzuweisen, dass die Phasenverschiebung des Lichts, von der optischen Weglänge durch das Objekt bestimmt ist, d.h. sowohl die geometrische Dicke des Objekt als auch der Brechungsindex spielt dabei eine Rolle. Um ein echtes geometrisches Höhenprofil aus der Phaseninformation zu erhalten muss der Brechungsindex der Probe bekannt sein. Im bevorzugten Anwendungsfall der Mikroskopie von Zellen kann man jedoch von einem weitestgehend konstanten und bekannten Brechungsindex ausgehen, wodurch die Phaseninformation direkt in ein Höhenprofil umgerechnet werden kann. Alternativ kann die Phaseninformation bei verschiedenen Wellenlängen aufgenommen werden und dadurch der Einfluss des Brechungsindex herausgerechnet werden.

Weiterhin ist zu beachten, dass die Phaseninformation nur auf einen Phasenwinkel von 360° eindeutig bestimmt ist. Eine gemessene Phasenverschiebung von beispielsweise 10° kann in Wirklichkeit einer Phasenverschiebung von 370°, 730°, usw. entsprechen. Der Eindeutigkeitsbereich ist demnach auf die Wellenlänge des Lichts beschränkt. Diese Limitierung ist im gewählten Anwendungsfall bei der mikroskopischen Betrachtung von Zellen ebenfalls vernachlässigbar, da biologische Zellen keine steilen Flanken aufweisen und somit die Phasenwerte der benachbarten Pixel als Anschlussbedingung zur Verfügung stehen.

Durch das erfindungsgemäße Verfahren kann eine gegenwärtig angewendete Vorgangsweise zum Erstellen eines Differenzialblutbilds markierungsfrei durchgeführt werden. Eine beispielhafte Vorgehensweise zum Erstellen eines Differenzialblutbilds umfasst zum Beispiel das Ermitteln einer Zellgröße, eines Reifungszustandes der Zelle, also eines Alters der Zelle, ein Kernplasmaverhältnis, das Erfassen von Zytoplasmaeinschlüssen und das Erfassen einer chromatinen Struktur.

Die Interferenzphasenmikroskopie, auch digitale holografische Mikroskopie (DHM) genannt, zeichnet sich dadurch aus, dass sie sowohl eine Intensität als auch eine Phase eines Objektes quantitativ in einer Aufnahme erfassen kann. Dafür wird nicht, wie in der Mikroskopie üblich, ein Abbild des Objekts erfasst, sondern eine Wellenfront, welche sich aus der Überlagerung einer Objekt- und einer Referenzwelle ergibt. Daraus kann mit Hilfe einer Zellanalyseeinrichtung, zum Beispiel eines Computers, eine Intensitäts- und Phaseninformation des Objekts rekonstruiert werden.

Diese Mikroskopie-Methode eignet sich für eine Untersuchung von biologischen Proben, da diese ohne weitere Probenvorbereitung im Wesentlichen transparent sind für sichtbares Licht und damit in einem herkömmlichen Mikroskop einen geringeren Kontrast aufweisen. Durch die Aufnahme der Phaseninformationen ist es möglich, die Morphologie der Zelle sehr präzise zu erfassen und mit Hilfe dieser Informationen eine Differenzierung der Zelle vorzunehmen.

Eine Differenzierung von zum Beispiel weißen Blutkörperchen basierend auf zum Beispiel einer rekonstruierten Phaseninformation von mehreren Zellen kann ausgeführt werden, ohne dass eine Färbung der Zellen nötig ist und dass dadurch mit frischen, nicht getrockneten Zellen gearbeitet werden kann. Dadurch werden unnötige Artefakte, welche durch unterschiedliche Handhabung der Proben während des Trocknens und Färbens entstehen, vermieden. Die Bilder sind dabei reproduzierbarer und leichter durch eine automatische rechnergestützte Bildverarbeitung unterscheidbar. Die Anzahl der Prozessschritte wird minimiert. Dadurch werden Kosten und Zeit eingespart und eine zuverlässige und schnellere Diagnostik ermöglicht.

In der Pre-Analytik kann so gegenüber der Hämatologie-Analysatoren auf den Einsatz von zum Beispiel Rounding Buffers verzichtet werden (das heißt hochverdünnte SDS-Pufferlösung). Eine solche würde zum Aufrunden von Zellen führen, die zwar für die Streulichtmessung beim Durchflusszytometer geeignet ist, aber gleichzeitig zu Artefakten führt.

Die Erfindung wird anhand der beigefügten Zeichnungen noch einmal durch konkrete Ausführungsbeispiele näher erläutert. Die gezeigten Beispiele stellen bevorzugte Ausführungsformen der Erfindung dar. Es zeigen:

FIG 1     eine Übersichtsansicht für AML Probe,
FIG 2     eine Übersichtsansicht für CML Probe,
FIG 3     eine Übersichtsansicht für ALL Probe,
FIG 4     eine Übersichtsansicht für CLL Probe,
FIG 5     eine Übersichtsansicht für OMF Probe,
FIG 6     eine Übersichtsansicht für gesunder Probe,

es sind jeweils PCA-Parameter aufgetragen.

Weiter zeigen:

FIG 7     Charakteristische Streumuster für Myeloblasten- und Lymphoblasten-Gates von AML Probe,
FIG 8     Charakteristische Streumuster für Myeloblasten- und Lymphoblasten-Gates von CML Probe,

FIG 9     Charakteristische Streumuster für Myeloblasten- und Lymphoblasten-Gates von ALL Probe,

FIG 10    Charakteristische Streumuster für Myeloblasten- und Lymphoblasten-Gates von CLL Probe,

FIG 11    Charakteristische Streumuster für Myeloblasten- und Lymphoblasten-Gates von OMF Probe,

FIG 12    Charakteristische Streumuster für Myeloblasten- und Lymphoblasten-Gates von gesunder Probe.

Weiter zeigen:

FIG 13    Unterscheidung von Monozyten,

FIG 14    neutrophilen Granulozyten, eosinophilen Granulozyten,

FIG 15    basophilen Granulozyten, Lymphozyten,

FIG 16    eosinophilen Granulozyten, Promyelozyten, Blasten, Megakaryozyten,

FIG 17    Metamyleozyten

anhand charakteristischer Streumuster in definierten Bereichen ("Gates"). Es sind jeweils PCA-Parameter (Val-X) bzw. eine Kombination aus physikalischen und PCA-Parametern aufgetragen.

Mit Hilfe des erfindungsgemäßen In-Vitro-Verfahrens kann ein markierungsfreies Bestimmen eines Zelltyps einer weißen Blutzelle in einer biologischen Probe erfolgen. Markierungsfrei bedeutet hier, dass die Zellen nicht durch beispielsweise Fluoreszenzfarbstoffe oder radioaktive Partikel markiert werden müssen. Eine biologische Probe kann dabei beispielsweise eine Probe tierischer oder menschlicher Blutzellen umfassen. Vorzugsweise handelt es sich dabei um eine Vollblutprobe, die zum Beispiel Blutzellen 10, z.B. Leukozyten, eosinophile Granulozyten oder basophile Granulozyten umfasst.

**In einem ersten experimentellen Beispiel** erfolgt die Unterscheidung von AML, CML, ALL, CLL und OMF anhand der jeweiligen charakteristischen Streumuster.

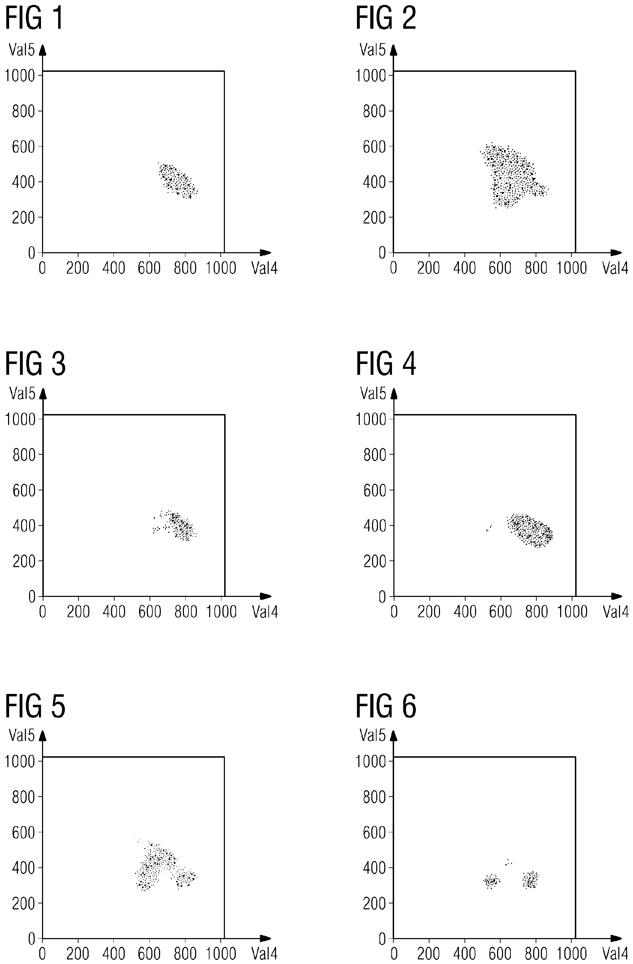

Fig. 1 bis Fig. 6 zeigen charakteristische Streumuster von AML (Fig.1), CML (Fig. 2), ALL (Fig. 3), CLL (Fig. 4), OMF (Fig. 5) und gesunder Probe (Fig. 6). "OMF" steht für Osteomyelofibrose, einer Unterart der Gruppe der Myeloproliferativen Neoplasien (MPN). Es werden die PCA-Parameter Val4 und Val5 (entsprechende Linearkombination siehe oben) für die Unterscheidung verwendet.

Die Unterscheidungskriterien für Proben von Patienten mit einer der verschiedenen Leukämien bzw. von gesunden Probanden lauten wie folgt.

Probe von gesundem Probanden: drei eindeutig separate Datenbereiche:

    1. Bereich: Val4 = 400-650, Val5 = 200-500,
    2. Bereich: Val4 = 600-750, Val5 = 400-600,
    3. Bereich. Val4 = 700-850, Val5 = 250-400.

Probe von Patient mit AML: nur ein durchgehender Datenbereich:

$$\mathtt{Val4 = 600{-}900,\ Val5 = 300{-}600.}$$

Probe von Patient mit CML: zusammenhängender Datenbereich der sich in 3 Bereiche unterteilen lässt:

    1. Bereich: kleine Population im Bereich von Val4 = 400-550, Val5 = 200-500,
    2. Bereich: dominante Hauptpopulation im Bereich von Val4 = 500-700, Val5 = 200-500,
    3. Bereich: kleine Population im Bereich von Val4 = 700-850, Val5 = 250-400.

Probe von Patient mit ALL: große Hauptpopulation mit 2 kleinen Nebenpopulationen:

    1. Bereich: dominante Hauptpopulation im Bereich von Val4 = 700-850, Val5 = 300-500,
    2. Bereich: kleine Population im Bereich von Val4 = 550-700, Val5 = 300-450,
    3. Bereich: kleine Population im Bereich von Val4 = 500-700, Val5 = 450-600.

Probe von Patient mit CLL: große Hauptpopulation mit einer kleinen Nebenpopulation:

    1. Bereich: dominante Hauptpopulation im Bereich von Val4 = 650-900, Val5 = 300-500,
    2. Bereich: kleine Population im Bereich von Val4 = 400-600, Val5 = 300-500.

Probe von Patient mit OMF: zusammenhängender Datenbereich der sich in 4 Bereiche unterteilen lässt:

1. Bereich: Val4 = 700-900, Val5 = 200-400,
2. Bereich: Val4 = 600-750, Val5 = 400-550,
3. Bereich: Val4 = 500-650, Val5 = 250-450
4. Bereich: kleine Population im Bereich von Val4 = 400-650, Val5 = 450-700.

**In einem zweiten experimentellen Beispiel** erfolgt die Unterscheidung von AML, CML, ALL, CLL und OMF anhand der jeweiligen charakteristischen Streumuster.

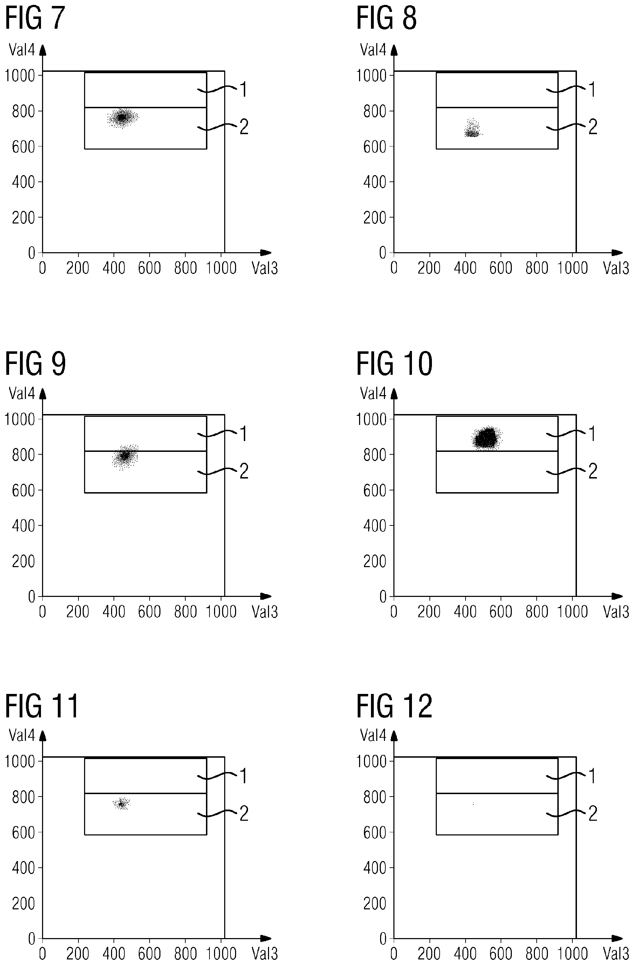

Fig. 7 bis 12 zeigen charakteristische Streumuster von AML (Fig. 7), CML (Fig. 8), ALL (Fig. 9), CLL (Fig. 10), OMF (Fig. 11) und gesunder Probe (Fig. 12). Es werden die PCA-Parameter Val3 und Val4 (entsprechende Linearkombination siehe oben) für die

Unterscheidung verwendet. Zwei große Bereiche werden definiert:

1. Bereich: Val4 = 235-920, Val5 = 576-816 (= Gate "Myeloblasts" (2)),
2. Bereich: Val4 = 235-920, Val5 = 816-1015 (= Gate "Lymphoblasts" (1)).

Die Unterscheidungskriterien für Proben von Patienten mit einer der verschiedenen Leukämien bzw. von gesunden Probanden lauten wie folgt.

Probe von gesundem Probanden: keine oder lediglich sehr geringe Anzahl an Datenpunkten in beiden Gates.

Probe von Patient mit AML: signifikante Population im 1. Bereich:

> 90% aller Datenpunkte im 1. Bereich,
< 10% aller Datenpunkte im 2. Bereich.

Probe von Patient mit CML: geringe Anzahl an Datenpunkten im 1. Bereich:

> 90% aller Datenpunkte im 1. Bereich,
< 10% aller Datenpunkte im 2. Bereich.

Probe von Patient mit ALL: signifikante Population erstreckt sich über 1. und 2. Bereich:

< 90% aller Datenpunkte im 1. Bereich,
> 10% aller Datenpunkte im 2. Bereich.

Probe von Patient mit CLL: signifikante Population im 2. Bereich:

< 10% aller Datenpunkte im 1. Bereich,
> 90% aller Datenpunkte im 2. Bereich.

Probe von Patient mit OMF: geringe Anzahl an Datenpunkten im 1. und 2. Bereich:

> 80% aller Datenpunkte im 1. Bereich,
< 20% aller Datenpunkte im 2. Bereich.

**In einem dritten experimentellen Beispiel** erfolgt die markierungsfreie Unterscheidung der 5 Haupttypen der weißen Blutzellen sowie weiterer Subtypen.

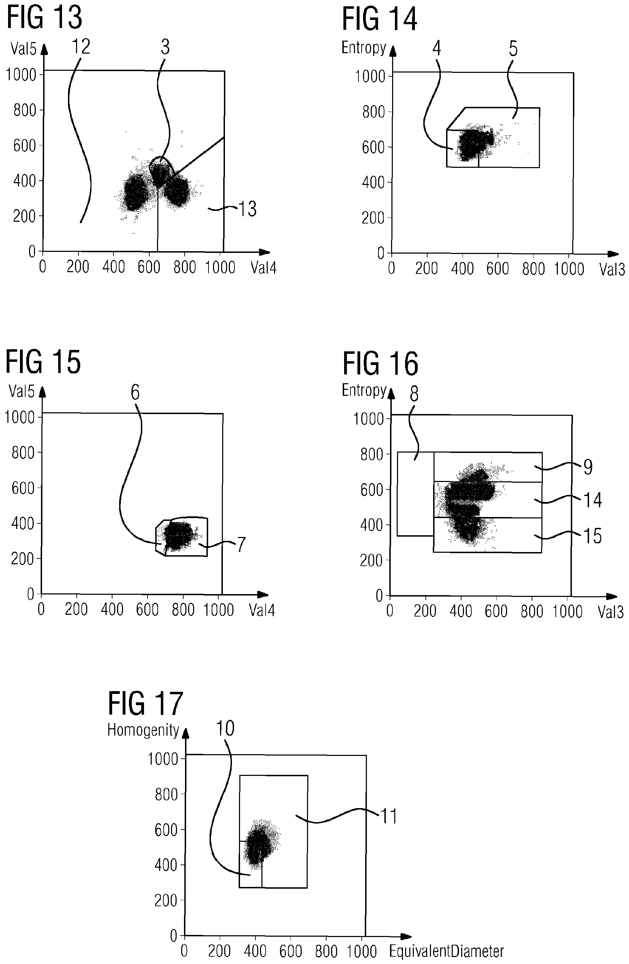

Fig. 13 bis 17 zeigen die markierungsfreie Unterscheidung der 5 Haupttypen der weißen Blutzellen sowie weiterer Subtypen anhand charakteristischer Streumuster in definierten Bereichen ("Gates"). Die Abkürzung "E & N & IG" (12) steht für Eosinophile, Neutrophile und unreife Granulozyten (siehe Fig. 13). Bei den unreifen Granulozyten ("immature granulocytes") handelt es sich in diesem Fall um Metamyelozyten und Myelozyten. Die folgenden Bereiche werden für die Unterscheidung der Zelltypen definiert (angegeben sind jeweils die verwendeten Parameter und die X/Y Koordinaten der definierten Bereiche(Gates)):

1. Bereich = Gate "Monocytes" (3): Zelltyp Monozyten werden wie folgt aufgrund der Werte von Val4 und Val5 unterschieden.

| Val4 | Val5 |
|------|------|
| 606 | 483 |
| 636 | 522 |
| 664 | 538 |
| 697 | 527 |
| 727 | 496 |
| 746 | 435 |
| 650 | 361 |
| 622 | 438 |

2. Bereich = Gate "Neutros" (4): Zelltyp Neutrophile werden wie folgt aufgrund der Werte von Val4 und Val5 unterschieden.

| Val4 | Val5 |
|------|------|
| 501 | 620 |
| 494 | 486 |
| 477 | 487 |
| 456 | 486 |
| 416 | 487 |
| 321 | 487 |
| 322 | 564 |
| 323 | 607 |
| 322 | 659 |
| 322 | 703 |
| 480 | 696 |

3. Bereich = Gate "Eos"(5): Zelltyp Eosinophile werden wie folgt aufgrund der Werte von Val4 und Entropy unterschieden.

| Val4 | Entropy |
|------|---------|
| 320 | 703 |
| 406 | 831 |
| 564 | 833 |
| 580 | 831 |
| 654 | 828 |
| 739 | 827 |
| 838 | 826 |
| 838 | 671 |
| 840 | 488 |
| 724 | 488 |
| 625 | 486 |
| 494 | 487 |
| 501 | 627 |

(fortgesetzt)

| Val4 | Entropy |
|------|---------|
| 479 | 697 |

4. Bereich = Gate "Basos"(6): Zelltyp Basophile werden wie folgt aufgrund der Werte von Val4 und Val5 unterschieden.

| Val4 | Val5 |
|------|------|
| 732 | 422 |
| 717 | 347 |
| 699 | 222 |
| 643 | 254 |
| 645 | 386 |
| 683 | 415 |

5. Bereich = Gate "Lymphos"(7): Zelltyp Lymphozyten werden wie folgt aufgrund der Werte von Val4 und Val5 unterschieden.

| Val4 | Val5 |
|------|------|
| 781 | 438 |
| 826 | 436 |
| 936 | 426 |
| 937 | 223 |
| 861 | 223 |
| 801 | 218 |
| 698 | 212 |
| 717 | 347 |
| 731 | 424 |

6. Bereich = Gate "Megakaryocytes" (8): Zelltyp Megakaryozyten werden wie folgt aufgrund der Werte von Val3 und Entropy unterschieden.

| Val3 | Entropy |
|------|---------|
| 33 | 233 |
| 332 | 805 |

7. Bereich = Gate "Promyelocytes" (9): Zelltyp Promyelozyten werden wie folgt aufgrund der Werte von Val3 und Entropy unterschieden.

| Val3 | Entropy |
|------|---------|
| 233 | 846 |
| 642 | 805 |

8. Bereich = Gate "Myelocytes" (10): Zelltyp Myelozyten werden wie folgt aufgrund der Werte von EquivalentDiameter und Homogenity unterschieden.

| EquivalentDiameter | Homogenity |
|---|---|
| 333 | 534 |
| 304 | 534 |
| 304 | 493 |
| 304 | 436 |
| 304 | 272 |
| 367 | 273 |
| 429 | 274 |
| 427 | 408 |
| 427 | 470 |
| 418 | 524 |
| 400 | 532 |
| 377 | 534 |
| 356 | 534 |

9. Bereich = Gate "Metamyelocytes" (11): Zelltyp Metamyelozyten werden wie folgt aufgrund der Werte von Equivalent-Diameter und Homogenity unterschieden.

| EquivalentDiameter | Homogenity |
|---|---|
| 304 | 534 |
| 301 | 907 |
| 680 | 900 |
| 684 | 273 |
| 428 | 273 |
| 426 | 372 |
| 426 | 468 |
| 420 | 524 |
| 402 | 532 |
| 379 | 534 |

**Patentansprüche**

1. In-vitro Verfahren zum markierungsfreien Bestimmen eines Zelltyps einer weißen Blutzelle in einer biologischen Probe, wobei eine Mikroskopiervorrichtung die Zelle abbildet und aus der Abbildung der Zelle mittels einer automatischen Bildanalyse physikalische Parameter der Zelle ermittelt werden, **dadurch gekennzeichnet, dass** anhand der physikalischen Parameter und anhand von Hauptkomponentenanalyse-Parametern (PCA-Parametern) der Zelltyp der weißen Blutzelle bestimmt wird, wobei die Hauptkomponentenanalyse-Parameter Linearkombinationen wenigstens eines Teils der physikalischen Parameter umfassen.

2. Verfahren nach Anspruch 1, wobei es sich bei dem bestimmten Zelltyp der weißen Blutzelle um einen der folgenden Haupttypen von weißen Blutzellen aus der Gruppe umfassend Monozyten, neutrophile Granulozyten, basophile Granulozyten, eosinophile Granulozyten und Lymphozyten handelt.

3. Verfahren nach einem der Ansprüche 1 oder 2, wobei es sich bei dem bestimmten Zelltyp um einen der Haupttypen von weißen Blutzellen und/oder einem Subtyp von weißen Blutzellen aus der Gruppe umfassend Myelozyten, Metamyelozyten, Promyelozyten, Blasten, Megakaryozyten, Plasmazellen, atypische Lymphozyten und Sezary Zellen handelt.

4. Verfahren nach einem der vorhergehenden Ansprüche, wobei anhand der Bestimmung des jeweiligen Zelltyps einer Vielzahl von weißer Blutzellen in einer Probe die jeweiligen Zellpopulationen charakterisiert werden und anhand der vorliegenden Zellpopulationen bestimmt wird, ob eine akute myeloische Leukämie (AML), eine akute lymphatische Leukämie (ALL), eine chronische myeloische Leukämie (CML) oder eine chronische lymphatische Leukämie (CLL) in dem Patient, von dem die Probe stammt, vorliegt.

5. Verfahren nach einem der vorhergehenden Ansprüche, wobei die physikalischen Parameter der Zelle Parameter aus der folgenden Gruppe umfassend umfasste Fläche der Zelle, Umfang der Zelle, Breite der Zelle, Höhe der Zelle, Verhältnis von Breite und Höhe der Zelle, Ähnlichkeit der geometrischen Form der Zelle mit einem Kreis, durchschnittlicher Radius der Zelle, Varianz des Radius der Zelle, Bedeckungsgrad der Zelle, äquivalenter Durchmesser entsprechend der umfassten Fläche der Zelle, optisches Volumen der Zelle, maximale optische Höhe der Zelle, minimale optische Höhe der Zelle, durchschnittliche optische Höhe der Zelle, Varianz der optischen Höhe der Zelle, Bikonkavität der Zelle, Sphärizität der Zelle, Verschiebung des Massenschwerpunkts der Zelle, Kontrast der Zelle, Verschiedenheit der Zelle, Homogenität der Zelle, Energie der Zelle, Entropie der Zelle umfassen.

6. Verfahren nach einem der vorhergehenden Ansprüche,
   wobei die Abbildung der Zelle ein Überlagern einer Objektwelle mit einer Referenzwelle, einem Aufnehmen eines resultierenden Interferogramms und/oder einer computerimplementierten mathematischen Rekonstruktion umfasst.

7. Verfahren nach einem der vorhergehenden Ansprüche, wobei es sich bei der Mikroskopiervorrichtung um ein Mikroskop zur Durchführung von digitaler holographischer Mikroskopie (DHM), Interferenzphasen-Mikroskopie oder quantitativer Phasen Mikroskopie handelt.

8. Verfahren nach einem der vorhergehenden Ansprüche, wobei die weißen Blutzellen in einer Durchflusszelle von der Mikroskopiervorrichtung abgebildet werden, wobei die Durchflusszelle bevorzugt einen Kanal mit rechteckigem oder quadratischem Querschnitt umfasst.

9. Verfahren nach Anspruch 8, wobei die weißen Blutzellen mittels laminarer Hüllströme, bevorzugt mittels vier laminarer Hüllströme, fokussiert werden.

10. Verfahren nach einem der vorhergehenden Ansprüche, wobei vor der Abbildung der weißen Blutzellen mittels der Mikroskopiervorrichtung rote Blutzellen mittels selektiver Lyse aus der Probe entfernt werden.

11. Verfahren nach einem der vorhergehenden Ansprüche, wobei das Verfahren mit einer ungefärbten und/oder ungetrockneten Zelle durchgeführt wird.

12. Verfahren nach einem der vorhergehenden Ansprüche, weiter umfassend den folgenden Schritt:

    - Phenotypisieren der Zelle mit einem Marker, und/oder
    - Exprimieren eines vorbestimmten Rezeptors

    zum weiteren Zuordnen der Zelle.

13. Verfahren nach einem der vorhergehenden Ansprüche, wobei das Verfahren anhand einer Vollblutprobe durchgeführt wird und/oder die biologische Probe Blutzellen, Monozyten, neutrophile Granulozyten, basophile Granulozyten, eosinophile Granulozyten und/oder Lymphozyten enthält.

14. Zellanalyseeinrichtung, die dazu eingerichtet ist, ein Verfahren nach einem der Ansprüche 1 bis 13 durchzuführen.

15. Mikroskopiervorrichtung zum markierungsfreien Bestimmen eines Zelltyps einer Zelle einer biologischen Probe, umfassend eine Zellanalyseeinrichtung gemäß Anspruch 14.

FIG 1

FIG 2

FIG 3

FIG 4

FIG 5

FIG 6

FIG 7

FIG 8

FIG 9

FIG 10

FIG 11

FIG 12

FIG 13

FIG 14

FIG 15

FIG 16

FIG 17

Europäisches
Patentamt

European
Patent Office

Office européen
des brevets

# EUROPÄISCHER RECHERCHENBERICHT

**Nummer der Anmeldung**

EP 18 16 2033

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | YAMPRI P ET AL: "White Blood Cell Classification based on the Combination of Eigen Cell and Parametric Feature Detection", 2006 1ST IEEE CONFERENCE ON INDUSTRIAL ELECTRONICS AND APPLICATIONS, 1. Mai 2006 (2006-05-01), Seiten 1-4, XP031026640, PISCATAWAY, NJ : IEEE SERVICE CENTER, US DOI: 10.1109/ICIEA.2006.257341 ISBN: 978-0-7803-9513-8 | 1-5, 10-15 | INV. G06K9/00 G06K9/62 G01N33/49 |
| Y | * das ganze Dokument * ----- | 6-9 | |
| X | DER-CHEN HUANG ET AL: "A computer assisted method for leukocyte nucleus segmentation and recognition in blood smear images", JOURNAL OF SYSTEMS & SOFTWARE, Bd. 85, Nr. 9, 8. April 2012 (2012-04-08), Seiten 2104-2118, XP028499919, ELSEVIER NORTH HOLLAND, NEW YORK, NY, US ISSN: 0164-1212, DOI: 10.1016/J.JSS.2012.04.012 [gefunden am 2012-04-18] * Punkt 1. * * Punkt 3.1. * * Punkt 3.3. * * Punkt 3.4. * * Punkt 5.4. * * Punkt 6. * * Abbildungen 2,3 * * Tabellen 2,4 * * Zusammenfassung * ----- -/-- | 1-5, 10-15 | RECHERCHIERTE SACHGEBIETE (IPC) G06K G01N G03H |

Der vorliegende Recherchenbericht wurde für alle Patentansprüche erstellt

| Recherchenort | Abschlußdatum der Recherche | Prüfer |
|---|---|---|
| Den Haag | 23. August 2018 | Moreno, Marta |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.....................................................
& : Mitglied der gleichen Patentfamilie, übereinstimmendes
    Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 18 16 2033

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | EP 3 220 130 A1 (SIEMENS HEALTHCARE GMBH [DE]) 20. September 2017 (2017-09-20)<br>* Absatz [0001] *<br>* Absatz [0011] - Absatz [0012] *<br>* Absatz [0025] - Absatz [0027] *<br>* Absatz [0031] *<br>* Absatz [0079] *<br>----- | 6-9 | |
| A | WO 2015/195609 A1 (SIEMENS HEALTHCARE DIAGNOSTICS [US]) 23. Dezember 2015 (2015-12-23)<br>* Absatz [0002] *<br>* Absatz [0007] *<br>* Absatz [0030] *<br>* Absatz [0042] *<br>----- | 1-4,7, 14,15 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 23. August 2018 | Moreno, Marta |

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 18 16 2033

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-08-2018

| Im Recherchenbericht angeführtes Patentdokument | Datum der Veröffentlichung | Mitglied(er) der Patentfamilie | Datum der Veröffentlichung |
|---|---|---|---|
| EP 3220130 A1 | 20-09-2017 | EP 3220130 A1<br>WO 2017157555 A1 | 20-09-2017<br>21-09-2017 |
| WO 2015195609 A1 | 23-12-2015 | CN 106462746 A<br>CN 108426994 A<br>EP 3155558 A1<br>EP 3364341 A1<br>JP 2017519985 A<br>JP 2018119969 A<br>US 2017132450 A1<br>US 2018144182 A1<br>WO 2015195609 A1<br>WO 2015195642 A1 | 22-02-2017<br>21-08-2018<br>19-04-2017<br>22-08-2018<br>20-07-2017<br>02-08-2018<br>11-05-2017<br>24-05-2018<br>23-12-2015<br>23-12-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8406498 B

**In der Beschreibung aufgeführte Nicht-Patentliteratur**

- GLCM Texture: A Tutorial v. 3.0 March 2017. University of Calgary, Marz 2017